# EUROPÄISCHE PATENTSCHRIFT

(11) **EP 0 759 475 B1**
(45) Veröffentlichungstag und Bekanntmachung des Hinweises auf die Patenterteilung: **25.08.1999**
(21) Anmeldenummer: 96112269.4
(22) Anmeldetag: 30.07.1996
(51) Int. Cl.: C12P 41/00, C12P 13/04

(54) **Verfahren zur Herstellung von (R)-tertiär-Leucin**
Method for the production of (R)-ter-leucine
Méthode pour la production de leucine-(R)-tertiaire

(30) Priorität: 09.08.1995 DE 19529211
(43) Veröffentlichungstag der Anmeldung: 26.02.1997
(73) Patentinhaber: Degussa-Hüls Aktiengesellschaft, 45764 Marl (DE)
(72) Erfinder: Bommarius, Andreas, Dr., 60323 Frankfurt (DE); Drauz, Karlheinz, Prof. Dr., 63579 Freigericht (DE); Kottenhahn, Matthias, Dr., 63579 Freigericht (DE)

(56) Entgegenhaltungen:
- EP-A- 0 141 223
- NL-A- 9 001 680
- TETRAHEDRON: ASYMMETRY (1995), 6(12), 2851-8 CODEN: TASYE3;ISSN: 0957-4166, 1995, XP002017509 BOMMARIUS, ANDREAS S. ET AL: "Synthesis and use of enantiomerically pure tert - Leucine"

## Beschreibung

Die Erfindung betrifft ein Verfahren zur Herstellung von (R)-tertiär-Leucin. (R)-tertiär-Leucin ist von Bedeutung in der asymmetrischen Synthese, wo es, insbesondere in reduzierter Form als (R)-tert-Leucinol, zur effizienten Induktion von Asymmetrie bei vielen chemischen Reaktionen dient (vgl. A. S. Bommarius et al., Tetrahedron Asymmetry, 1995).

Im Gegensatz zur enantiomeren Form, dem (S)-tertiär-Leucin, das z. B. durch enzymatische reduktive Aminierung zugänglich ist ( a) C. Wandrey und B. Bossow, Biotechnol. Bioind. 1986, Band 3, Seiten 8 - 13, b) V. Kragl et al., Chem. Ing. Techn. 1992, Band 64, Seiten 499 - 509), ist (R)-tertiär-Leucin bis jetzt nur schwer zugänglich. So reagiert z. B. eine Acylase nur äußerst langsam mit N-Acetyl-(R,S)-tert-Leucin (vgl. H. K. Chenault, J. Dahmer und G. M. Whitesides, J. Amer. Chem. Soc. 1989, 111, 6354 - 64), so daß die Isolierung von (R)-tertiär-Leucin aus dem nicht hydrolysierten Acetyl-(R)-tert-Leucin sehr aufwendig ist und mit einer geringen Raum/Zeit-Ausbeute verläuft.

Aufgabe der Erfindung ist demnach, ein neues Verfahren zur Herstellung von (R)-tertiär-Leucin zur Verfügung zu stellen, das die o. g. Nachteile nicht aufweist.

Diese Aufgabe wird gemäß Anspruch 1 gelöst, indem man tertiär-Butylhydantoin mit einer (R)-Hydantoinase umsetzt und anschließend das entstandene N-Carbamoyl-D-tertiär-Leucin entweder durch eine erneute Enzymreaktion mit Carbamoylase oder durch Versetzung mit Nitrit zum (R) -tertiär-Leucin weiterreagieren läßt.

Die weiteren Ansprüche 2 bis 5 stellen bevorzugte Ausführungsformen dar.

(R)-Hydantoinasen sind im Grunde aus a) IT 1109506, 1978; b) R. Olivieri, E. Fascetti, L. Angelini und L. Degen, Enzyme and Microbial Technology, 1979, Band 1, Seiten 201 - 204; c) H. Yamada et al., J. Ferment. Technol. 1978, 56, 484ff.; d) C. Syldatk, et al., J. Biotechnol. 1990, 14, 345ff.; e) O. Keil, M. Schneider und J. P. Razor, Tetrahedron Asymmetry, 1995, 6, 1257 - 60;
f) EP 0 643 133 A2; g) NL 9001680 bekannt. In keinem Fall wurde jedoch die Umsetzung von tert.-Butylhydantoin zu N-Carbamoyl-(R)-tert.-Leucin und die weitere Umsetzung zu (R)-tertiär-Leucin beschrieben.

Überraschenderweise wurde nun gefunden, daß tert.Butylhydantoin von der (R)-Hydantoinase aus Escherichia coli vollständig zu N-Carbamoyl-(R)-tertiär-Leucin umgesetzt wird. Die weitere Umsetzung zu (R)-tertiär-Leucin kann mit Hilfe einer erneuten Enzymreaktion mit einer Carbamoylase erfolgen oder durch eine Weiterreaktion in Gegenwart von Nitrit.

Im erfindungsgemäßen Verfahren wird dabei (R,S)-tert.Butylhydantoin oder (S)-tert.Butylhydantoin in einem geeigneten Lösungsmittel, wie beispielsweise Wasser, bei pH-Werten zwischen 6,0 und 11, bevorzugt zwischen 7,5 und 10,5, besonders bevorzugt zwischen 8,5 und 10, und einer Temperatur zwischen 0 °C und 80 °C, bevorzugt zwischen 25 °C und 60 °C, besonders bevorzugt zwischen 40 °C und 50 °C, mit einer (R)-spezifischen Hydantoinase ggf. unter Zugabe von Hilfsstoffen, wie Metallionen, beispielsweise Mn²⁺-Ionen, bei konstantem pH umgesetzt. Nach dem Ende der Reaktion wird das entstandene Produkt N-Carbamoyl-(R)-tertiär-Leucin durch Feinfiltration (bei immobilisierten Enzymen) oder durch Ultrafiltration (bei löslichen Enzymen) vom Biokatalysator abgetrennt. Im nächsten Schritt wird in an sich bekannter Weise (C. Syldatk, A. Läufer, R. Müller und H. Höke, Adr. Biochem. Eng. / Biotechnol. 1990, Vol. 41, S. 29 ff. und darin zitierte Literatur) N-Carbamoyl-(R)-tertiär-Leucin mit Hilfe einer D-Carbamoylase zu (R)-tertiär-Leucin umgesetzt oder N-Carbamoyl-(R)-tertiär-Leucin wird in saurer wäßriger Lösung, wie beispielsweise HCl-saurerwäßriger Lösung, bei Temperaturen zwischen 0 und 30 °C durch Zugabe von Nitrit, wie beispielsweise Natriumnitrit, entcarbamoyliert und das entstandene Produkt durch Ionenaustauschchromatographie vom Salz befreit. Das Eluat wird mit Aktivkohle geklärt, das entstandene (R)-tertiär-Leucin durch Einengen des Lösungsmittels ausgefällt und getrocknet.

Vorteilhafterweise kann im erfindungsgemäßen Verfahren sowohl (R,S)-tertiär-Butylhydantoin als auch (S)-tertiär-Butylhydantoin als Ausgangsstoff verwendet werden, da die verwendeten Hydantoine unter den Reaktionsbedingungen racemisieren, so daß zu N-Carbamoyl-(R)-tertiär-Leucin abreagiertes (R)-tertiär-Butylhydantoin durch Racemisierung nachgebildet wird und der Umsatz so bis zu 100 % betragen kann.

Die verwendeten (R)-spezifischen Hydantoinasen können sowohl in löslicher Form als auch immobilisiert vorliegen. Besonders bevorzugt sind R-Hydantoinasen, wie in den Literaturstellen e) und f) beschrieben.

In den folgenden Beispielen soll die Erfindung näher erläutert werden, ist aber nicht auf diese beschränkt.

### Herstellung von (R)-tertiär-Leucin:

### Beispiel 1

5 g (32 mmol) tert.-Butylhydantoin werden mit 63,1 mg (0,5 mmol) Manganchlorid und 63,0 mg (0,5 mmol) Natriumsulfit in 1000 ml Wasser gelöst, auf pH 8,5 und 50 °C eingestellt und 1,5 kU (R)-Hydantoinase (immobilisiert, D-Hyd 1) der Firma Boehringer Mannheim zugegeben. Nach 8 Tagen ist der Umsatz vollständig. Die Reaktionslösung wird auf 300 ml eingeengt und der pH-Wert mit konzentrierter Schwefelsäure auf 0 eingestellt. Anschließend werden innerhalb von sechs Stunden bei guter Rührung 2,43 g (x mmol) Natriumnitrit in wäßriger Lösung zugetropft. Nach 24 Stunden war der Umsatz zu tert-Leucin vollständig. Die Mischung wird auf pH 7 eingestellt und durch Ionenaustauschchromatographie an stark saurem Harz von Salzen befreit. Die Aminosäure wird durch Verwendung von 5 %igem Ammoniak eluiert, der Ammoniak durch Vakuumdestillation bei 45 °C entfernt, bis der pH-Wert 7,5 beträgt. Zur Entfärbung wird mit 4 g A-Kohle geklärt, die Kohle abfiltriert und die geklärte Lösung bis zur Trockne eingeengt.
Ausbeute: 3,568 g (85,5 % der Theorie).

### Beispiel 2

Es wird genauso verfahren wie im Beispiel 1, nur daß der pH-Wert der Reaktionslösung mit (R)-Hydantoinase 9,5 beträgt. Vollständiger Umsatz war in vier Tagen erreicht statt in neun. Es wurde wie oben beschrieben zur Aminosäure entcarbamoyliert und das Aminosäure-Produkt aufgearbeitet.
Ausbeute: 3,412 g (81,8 % der Theorie)

Der Identitätsnachweis erfolgte durch spektroskopische Beweisführung.

## Patentansprüche

1. Verfahren zur Herstellung von (R)-tertiär-Leucin,
**dadurch gekennzeichnet,**
daß man tertiär-Butylhydantoin mit einer (R)-Hydantoinase umsetzt und anschließend das entstandene N-Carbamoyl-D-tertiär-Leucin entweder durch eine erneute Enzymreaktion mit Carbamoylase oder durch Versetzung mit Nitrit zum (R)-tertiär-Leucin weiterreagieren läßt.

2. Verfahren nach Anspruch 1,
**dadurch gekennzeichnet,**
daß eine (R)-Hydantoinase aus Escherichia coli verwendet wird.

3. Verfahren nach Anspruch 1,
**dadurch gekennzeichnet,**
daß die Hydantoinase immobilisiert vorliegt.

4. Verfahren nach Anspruch 1,
**dadurch gekennzeichnet,**
daß der pH-Wert der Reaktion zwischen 7,5 und 10,5 liegt.

5. Verfahren nach Anspruch 4,
**dadurch gekennzeichnet,**
daß der pH-Wert der Reaktion zwischen 8,5 und 10 liegt.

6. Verfahren nach Anspruch 1,
**dadurch gekennzeichnet,**
daß die Temperatur zwischen 25 °C und 60 °C liegt.

7. Verfahren nach Anspruch 6,
**dadurch gekennzeichnet,**
daß die Temperatur zwischen 40 °C und 50 °C liegt.

## Claims

1. Process for the preparation of (R)-tert.-leucine,
characterised in that
tert.-butylhydantoin is reacted with an (R)-hydantoinase, followed by further reacting the resulting N-carbamoyl-D-tert.-leucine to (R)-tert.-leucine either by another enzyme reaction, with carbamoylase, or by the addition of nitrite.

2. Process according to Claim 1,
characterised in that
an (R)-hydantoinase from Escherichia coli is employed.

3. Process according to Claim 1,
characterised in that
the hydantoinase is present in immobilised form.

4. Process according to Claim 1,
characterised in that
the pH of the reaction is between 7.5 and 10.5.

5. Process according to Claim 4,
characterised in that
the pH of the reaction is between 8.5 and 10.

6. Process according to Claim 1,
characterised in that
the temperature is between 25°C and 60°C.

7. Process according to Claim 6,
characterised in that
the temperature is between 40°C and 50°C.

## Revendications

1. Procédé de fabrication de leucine-(R)-tertiaire, caractérisé en ce qu'
on fait réagir la terbutylhydantoïne avec une (R)-hydantoinase et on laisse ensuite réagir la N-carbamoyl-D-leucine-tertiaire qui s'est formée soit par une réaction enzymatique renouvelée avec une carbamoylase soit par mélange avec du nitrite en leucine-(R)-tertiaire.

2. Procédé selon la revendication 1,
caractérisé en ce qu'
on utilise une (R)-hydantoïnase provenant d'Escherichia coli.

3. Procédé selon la revendication 1,
caractérisé en ce que
l'hydantoinase se présente sous forme immobilisée.

4. Procédé selon la revendication 1,
caractérisé en ce que
la valeur du pH de la réaction se situe entre 7,5 et 10,5.

5. Procédé selon la revendication 4
caractérisé en ce que
la valeur du pH de la réaction se situe entre 8,5 et 10.

6. Procédé selon la revendication 1,
caractérisé en ce que
la température se situe entre 25 et 60°C.

7. Procédé selon la revendication 6,
caractérisé en ce que
la température se situe entre 40°C et 50°C.
